(19) Europäisches Patentamt
European Patent Office
Office européen des brevets

(11) Veröffentlichungsnummer: **0 225 614**
**A2**

(12) # EUROPÄISCHE PATENTANMELDUNG

(21) Anmeldenummer: **86116911.8**

(51) Int. Cl.⁴: **C07D 239/46**, C07D 239/50

(22) Anmeldetag: **04.12.86**

(30) Priorität: **06.12.85 CH 5215/85**

(43) Veröffentlichungstag der Anmeldung:
**16.06.87 Patentblatt 87/25**

(84) Benannte Vertragsstaaten:
**AT BE CH DE ES FR GB IT LI LU NL SE**

(71) Anmelder: **LONZA AG**

**Gampel/Wallis(CH)**

(72) Erfinder: **O'Murchu, Colm, Dr.**
**Gebreitenweg 4 B**
**Visp (Kanton Wallis)(CH)**

(74) Vertreter: **Weinhold, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**D-8000 München 40(DE)**

(54) **Verfahren zur Herstellung von 2-substituierten 5-Nitroso-4,6-diamino-pyrimidinen.**

(57) Beim Verfahren zur Herstellung von 2-substituierten 5-Nitroso-4,6-diamino-pyrimidinen wird Malonsäuredinitril und ein Amidin in Gegenwart eines Nitritsalzes nitrosiert, wobei sich das entsprechende Amidinsalz von Isonitroso-malonitril bildet. Dieses wird dann in Gegenwart eines aprotischen polaren Lösungsmittels in basischem Milieu durch Wärmebehandlung zum Endprodukt umgesetzt.

EP 0 225 614 A2

# Verfahren zur Herstellung von 4-substituierten 5-Nitroso-2,6-diamino-pyrimidinen

Es ist bekannt, dass sich das 5-Nitroso-2,4,6-triamino-pyrimidin aus Malonsäuredinitril und einem Guanidinsalz herstellen lässt.

Durch Kondensation von Malonsäuredinitril und Guanidin-hydrochlorid bzw. -nitrat in Gegenwart von Natriumalkoholat in alkoholischer Lösung erhält man 2,4,6-Triamino-pyrimidin in wässiger Ausbeute - [W.Traube, Ber. 37, 4544 (1904); H.Sato et al. J.Chem.Soc.Japan Pure Chem.Sect. 72, 866 (1951), Chem.Abstr. 47, 5946 (1953)]. Dieses Pyrimidin wird dann zu 5-Nitroso-2,4,6-triamino-pyrimidin mit salpetriger Säure nitrosiert [M.F.Mallette et al, J.Am.Chem.Soc. 69, 1814 (1947)]. Diese Verfahren haben den Nachteil, dass sie zu umständlich sind für die Herstellung von grösseren Mengen, und dass die Ausbeute an 5-Nitroso-2,4,6-triamino-pyrimidin höchstens 75 bis 78%, bezogen auf Malonsäuredinitril, beträgt.

Es wurde versucht, dieses Verfahren zu vereinfachen, indem man das Zwischenprodukt 2,4,6-Triamino-pyrimidin nicht isolierte (CH-PS 630 616). Diesem Verfahren haften aber immer noch verschiedene Nachteile an, es muss teures Natriumalkoholat verwendet werden, pro Mol eingesetztes Malonsäuredinitril fallen mindestens zwei Mol Salz (NaCl und Na-acetat) an, es muss mit relativ verdünnten Reaktionslösungen gearbeitet werden (ca. 2 l Lösungmittel pro Mol Produkt), und schliesslich gestaltet sich die Wiederaufarbeitung der Lösungsmittel als sehr schwierig, da es sich um ein 4-Komponenten-Lösungsmittelgemisch (Methanol, Aethanol, Eisessig, Wasser und Nebenprodukte) handelt.

Ein anderer Weg, 5-Nitroso-2,4,6-triamino-pyrimidin herzustellen, ist in E.C.Taylor, O.Vogl and C.C.Cheng, J.Am.Chem.Soc. 81, 2442 (1959) beschrieben. Durch Erhitzen des Kaliumsalzes von Isonitroso-malonitril mit Guanidin-carbonat in Dimethylformamid erhält man 5-Nitroso-2,4,6-triamino-pyrimidin in 88% Ausbeute. Da das Kaliumsalz aus dem Silbersalz von Isonitroso-malonitril hergestellt wird, kommt dieses Verfahren für eine grosstechnische Produktion nicht in Frage.

In der FR-PS 1 364 734 wird ein Verfahren beschrieben, in dem zuerst das Malonsäuredinitril in wässriger Essigsäure-Lösung mit Natriumnitrit nitrosiert, dann die gebildete Isonitrosomalonitril-Lösung mit Guanidin-carbonat behandelt wird, wobei $CO_2$ entweicht und das Guanidinsalz von Isonitroso-malonitril ausfällt. Diese Salz-Suspension wird dann auf ca. 0°C abgekühlt, filtriert und das Guanidinsalz von Isonitrosomalonitril getrocknet. Das Salz wird dann in Dimethylformamid nach Zugabe von $K_2CO_3$ unter Rückfluss erhitzt, um die Isomerisierung zu 5-Nitroso-2,4,6-triamino-pyrimidin zu vollziehen.

Dieses Verfahren stellt einen gewissen Fortschritt gegenüber dem Verfahren nach Taylor dar, weist aber noch mehrere Nachteile auf. So muss die Essigsäure im Ueberschuss verwendet werden (10% laut Beispiel). Dieser Ueberschuss muss mit Guanidin-carbonat neutralisiert werden, um das Guanidinsalz von Isonitroso-malonitril vollständig zu bilden.

Das Kühlen von wässriger Suspension auf ca. 0°C ist technisch mit Schwierigkeiten verbunden, weil eine Kruste von Eis an der inneren Seite des Reaktionsgefässes gebildet wird. Beim Kühlen auf weniger tiefe Temperaturen wird das Salz nicht vollständig ausgefällt.

Das Trocknen des Guanidinsalzes von Isonitroso-malonitril ist aus sicherheitstechnischen Gründen riskant.

Bei der Behandlung von roher Isonitroso-malonitril-Lösung mit Guanidin-carbonat wird ein Aequivalent $CO_2$ freigesetzt. Das Reaktionsgemisch hat daher die Neigung, während dieses Arbeitsganges zu - schäumen, so dass das Reaktionsgefäss nicht optimal ausgenutzt werden kann.

Aus der EP-Anmeldung EP 115 325 ist es weiterhin bekannt 2-substituierte-5-Nitroso-4,6-diaminopyrimidine durch Umsetzung von Malonsäuredinitril mit Amidinen in Gegenwart eines Nitritsalzes in saurem Milieu und in Wasser oder Alkohol als Lösungsmittel zum Amidinsalz des Isonitrosomalonitrils und durch Weiterüberführung dieses Salzes in Dimethylformamid in basischem Milieu durch Wärmebehandlung zu erhalten. Ein grosser Nachteil dieses Verfahrens ist das mit der Verwendung von Dimethylformamid auftretende Dimethylnitrosoamin.

Aufgabe der vorliegenden Erfindung ist es, diese Nachteile zu vermeiden und ein Verfahren vorzuschlagen, das es ermöglicht, auf einfache, wirtschaftliche und besonders auf risikolose Weise und in hoher Ausbeute 2-substituierte 5-Nitroso-4,6-diamino-pyrimidine herzustellen.

Erfindungsgemäss wird das mit einem Verfahren nach Anspruch 1 erreicht.

Bei speziellen Ausführungsformen der vorliegenden Erfindung bedeutet dabei der Substituent R in dem eingesetzten Amidin (und entsprechend auch als 2-Substituent der erhaltenen 5-Nitroso-4,6-diamin-pyridine) insbesondere $C_1$-$C_4$-Alkyl, $C_1$-$C_4$-Alkylthio, Benzyl, Phenyl, Amino bzw. $C_1$-$C_4$-Alkylamino.

Auf bekannte Weise wird darin das Malonsäuredinitril zweckmässig in Wasser oder Alkohol als Lösungsmittel mit einem Amidin in saurem Milieu in Gegenwart eines Nitritsalzes nitrosiert, wobei sich direkt das Amidinsalz von Isonitroso-malonitril bildet dieses weiter ohne es zu isolieren, basisch und nach Zugabe eines polaren aprotischen Lösungsmittels nach Entfernen des Wassers oder des Alkohols wärmebehandelt, wobei sich das entsprechende 2-substituierte 5-Nitroso-4,6-diamino-pyrimidin bildet. Erfindungsgemäss werden als polares aprotisches Lösungsmittel Dimethylsulfoxid, N-Methyl-2-pyrrolidon, N,N-Dimethylacetamid, Hexamethylphosphorsäuretriamid, 1,3-Dimethyl-2-oxo-hexahydropyrimidin oder Tetrahydro-thiophen-1,1-dioxid usw. eingesetzt. Bevorzugt wird Dimethylsulfoxid eingesetzt. Neben diesen Lösungsmitteln können auch Pyridinbasen, wie z.B. α-Picolin, β-Picolin, γ-Picolin, 2-Methyl-5-ethyl-pyridin, Lutidine eingesetzt werden.

Die Reaktion folgt der allgemeinen Formel

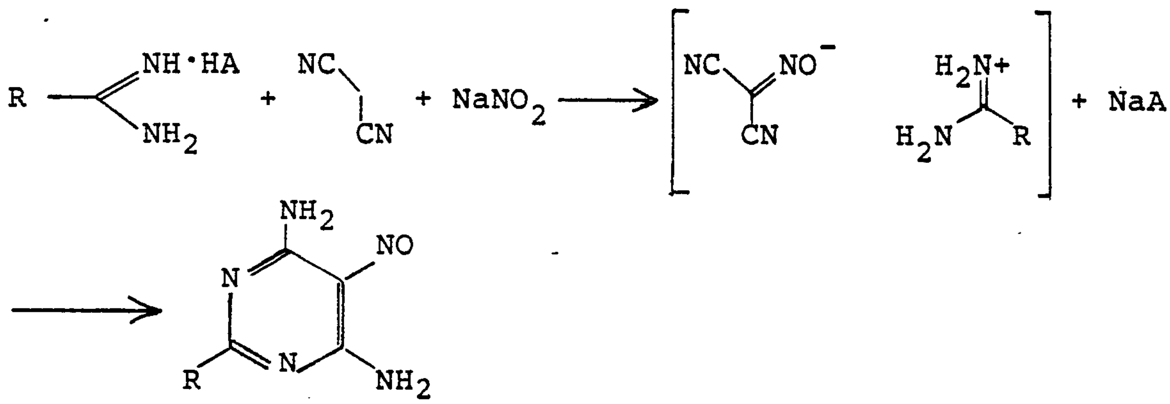

wobei A = Cl, 1/2 $SO_4$, $HSO_4$, $NO_3$, Acetat oder Phosphat und R = Aryl, Alkyl, Alkylthio, Amino, substituiertes Amino oder Arylalkyl bedeutet.

Beispiele für Amidine sind Acetamidinhydrochlorid, Benzamidinhydrochlorid, S-Methylisothioharnstoffsulfat und Guanidinhydrochlorid. Bevorzugtes Amidin ist das Guanidinhydrochlorid.

Als Nitritsalz können die Alkali-oder Erdalkalimetallnitrite, vorzugsweise Natriumnitrit, verwendet werden. Unter einem sauren Milieu wird ein pH-Wert von kleiner als 6,9 verstanden.

Bezüglich der Mengenverhältnisse werden zweckmässig 0,1 bis 1,1 Mol Nitrit, vorzugsweise 1,0 Mol bis 1,02 Mol Nitrit, pro Mol Malonsäuredinitril angewendet.

Die Lösungsmittelmenge für die erste Reaktionsstufe ist nicht kritisch und beträgt zweckmässig 200 bis 2000 ml pro Mol Malonsäuredinitril. Bevorzugt werden 300 bis 400 ml Lösungsmittel pro Mol Malonsäuredinitril eingesetzt. Die Reaktionstemperatur für die erste Stufe liegt zweckmässig zwischen 10 und 50°C. Nach einer Reaktionszeit von ungefähr 0,5 bis 15 Stunden und nach üblicher Aufarbeitung, durch z.B. Filtration und anschliessende Trocknung, kann dann das Amidinsalz des Isonitrosomalonitrils erhalten werden.

Das Amidinsalz des Isonitrosomalonitrils kann aber auch ohne Isolation direkt durch Zugabe von Dimethylsulfoxid als Lösungsmittel in der zweiten Stufe eingesetzt werden. In der zweiten Reaktionsstufe, mit Dimethylsulfoxid als Lösungsmittel, können pro Mol Malonsäuredinitril 100 bis 2000 ml, bevorzugt 300 bis 800 ml, Lösungsmittel eingesetzt werden.

Das notwendige basische Milieu kann zweckmässig durch Zugabe von Natriumhydroxid, Natriumcarbonat, Kaliumhydroxid, Kaliumcarbonat, substituierte Pyridine erreicht werden.

Bevorzugte Basen sind Natriumcarbonat und Kaliumcarbonat.

Für die abschliessende Wärmebehandlung wird das Reaktionsgemisch zweckmässig auf Temperaturen von 100 bis 180°C, vorzugsweise von 130 bis 160°C während zweckmässig 0,25 bis 6 Stunden, vorzugsweise 1 bis 4 Stunden erhitzt.

In einer vorzugsweisen Ausführungsform wird 5-Nitroso-2,4,6-triamino-pyrimidin hergestellt; indem Malonsäuredinitril und Guanidin-hydrochlorid bei einem pH unter 6,9 in Gegenwart von Natriumnitrit zum Guanidinsalz des Isonitroso-malonitrils umgesetzt und wird dieses durch Erhitzen auf 150°C in Dimethylsulfoxid und in Gegenwart von $Na_2CO_3$ das Guanidinsalz des Isonitroso-malonitrils zum 5-Nitroso-2,4,6-triamino-pyrimidin umgesetzt wird.

Die nach beendeter Reaktion vorliegenden 2-substituierten 5-Nitroso-4,6-diamino-pyrimidine können auf übliche Weise durch Filtration oder Zentrifugieren abgetrennt, mit Wasser gewaschen und getrocknet werden. Die 2-substituierten 5-Nitroso-2,4,6-diaminopyrimidine, insbesonderer Weise das 5-Nitroso-2,4,6-triamino-pyrimidin sind vielseitige Zwischenprodukte, z.B. zur Herstellung von Medikamenten, wie Triamteren, Methothrexat, und zur Herstellung von Farbstoff-Komponenten, wie 2,4,5,6-Tetraamino-pyrimidin.

Beispiel 1

Zu einer Suspension von 66 g Malonsäuredinitril und 100 g Guanidinhydrochlorid in 200 g Wasser tropfte man bei pH 4 und bei Raumtemperatur eine Lösung von 70 g Natriumnitrit in 120 ml Wasser hinzu. Nach 4 Stunden Rühren bei Raumtemperatur wurden 21 g Natriumcarbonat und 770 g eines polar aprotischen Lösungsmittels zugegeben und das Wasser bei vermindertem Druck abdestilliert. Anschliessend wurde das Reaktionsgemisch während 3 Stunden bei 140 bis 150°C erhitzt, wobei Isomerisierung zu 5-Nitroso-2,4,6-triamino-pyrimidin stattfand. Nach beendeter Reaktion wurden 750 g Wasser zugegeben und das Produkt wurde abfiltriert und mit Wasser gewaschen. Die Ergebnisse der Versuche in den verschiedenen polar aprotischen Lösungsmitteln sind in Tabelle 1 zusammengestellt.

| Beispiel | Lösungsmittel | Produkt-Menge g | Ausbeute % |
|---|---|---|---|
| 1 - 1 | Dimethylsulfoxid | 138 | 89,6 |
| 1 - 2 | Dimethylacetamid | 118 | 76,6 |
| 1 - 3 | N-Methyl-2-pyrrolidon | 142 | 92,2 |
| 1 - 4 | Tetrahydro-thiophen-1,1-dioxid | 147 | 95,5 |
| 1 - 5 | 3-Picolin | 130 | 84,4 |
| 1 - 6 | 4-Picolin | 144 | 93,5 |
| 1 - 7 | 2-Methyl-5-ethylpyridin | 151 | 98,0 |
| (1 - 8* | Dimethylformamid | 128 | 83,1) |
| 1 - 9 | 2-Methylglutarodinitril | 111 | 72,1 |
| 1 - 10 | Cyclohexanon | 86 | 55,8 |
| 1 - 11 | Hexamethylphosphorsäure-triamid | 141 | 91,6 |
| 1 - 12 | 1,3-Dimethyl-2-oxo-hexahydropyrimidin | 145 | 94,2 |

*Vergleichsversuch

Beispiel 2

Zu einer Suspension von 33 g Malonitril und 52 g Acetamidinhydrochlorid in 100 g Wasser tropfte man bei pH 4 und bei Raumtemperatur eine Lösung von 37,5 g Natriumnitrit in 60 g Wasser hinzu. Nach einer Nachreaktion von 4 Stunden wurde die Reaktionsmischung auf 0°C gekühlt und das Produkt abfiltriert. Man erhielt das Acetamidinsalz von Isonitroso-malonitril mit dem Smp. 142 bis 143°C (Zersetzung) in fast quantitativer Ausbeute (84% isoliert).

Beispiel 3

Zu einer Suspension von 13,2 g Malonitril und 32 g Benzamidinhydrochlorid in 25 g Wasser tropfte man bei pH 3 bis 5 und bei 20°C eine Lösung von 14 g Natriumnitrit in 25 g Wasser hinzu. Nach einer Nachreaktion von 5 Stunden und Abkühlen auf 0°c wurde das Reaktionsprodukt abgenutscht und getrocknet. Man erhielt das Benzamidinsalz von Isonitroso-malonitril mit dem Smp. 150°C (Zersetzung) in fast quantitativer Ausbeute (94% isoliert).

Beispiel 4

Zu einer Suspension von 33 g Malonitril und 70 g S-Methylisothioharnstoff-sulfat in 100 ml Wasser tropfte man bei pH 4 und bei Raumtemperatur eine Lösung von 35 g Natriumnitrit in 60 ml Wasser hinzu. Nach einer Nachreaktion von 5 Stunden wurde das Reaktionsgemisch auf 4°C gekühlt und abfiltriert.

Nach dem Trocknen erhielt man das S-Methylisothiouroniumsalz von Isonitroso-malonitril in sehr hoher Ausbeute (76% isoliert), Smp. 123 bis 124°C (Zersetzung).

Beispiel 5

Zu einer Suspension von 66 g Malonitril und 97 g Guanidinhydrochlorid in 120 ml Wasser tropfte man bei pH 4 und bei Raumtemperatur eine Lösung von 70 g Natriumnitrit in 120 ml Wasser hinzu. Nach 4 Stunden Rühren wurde auf 0°C gekühlt und abfiltriert.

Nach dem Trocknen im Vakuum erhielt man das Guanidinsalz von Isonitroso-malonitril in fast quantitativer Ausbeute (84% isoliert), Smp. 160 bis 161°C (Zersetzung).

Die weitere Umsetzung dieser Amidin-Salze des Isonitroso-malonitrils aus den Beispielen 2 bis 5 zu den entsprechenden 2-substituierten 5-Nitroso-4,6-diamino-pyrimidinen kann, wie in der Literatur E.C.Taylor et al, J.Am.Chem.Soc. 81, 2442 (1959) beschrieben, durchgeführt werden.

## Ansprüche

1. Verfahren zur Herstellung von 2-substituierten 5-Nitroso-4,6-diamino-pyrimidinen der allgemeinen Formel

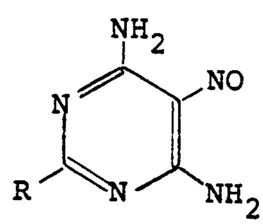

wobei R = Aryl, Alkyl, Alkylthio, Amino, substituiertes Amino oder Arylalkyl bedeutet durch Umsetzung von Malonsäuredinitril mit einem Amidin der allgemeinen Formel

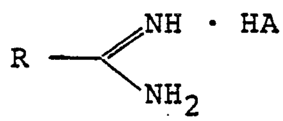

wobei A = Cl, 1/2 $SO_4$, $HSO_4$, $NO_3$, Acetat oder Phosphat bedeutet und R die oben angegebene Bedeutung hat, in Wasser oder Alkohol in saurem Milieu in Gegenwart eines Nitritsalzes zum entsprechenden Amidinsalz des Isonitroso-malonitrils und weitere Ueberführung des Amidinsalzes des Isonitroso-malonitrils in einem polaren aprotischen Lösungsmittel durch Wärmebehandlung zum Endprodukt, dadurch gekennzeichnet, dass als polares aprotisches Lösungsmittel für die Umsetzung des Amidinsalzes des Isonitroso-malonitrils zum entsprechenden 2-substituierten 5-Nitroso-4,6-diamino-pyrimidin Dimethylsulfoxid, N-Methyl-2-pyrrolidon, N,N-Dimethyl-acetamid, Hexamethylphosphorsäuretriamid, 1,3-Dimethyl-2-oxo-hexahydropyrimidin, Tetrahydro-thiophen-1,1-dioxid, 2-Methylglutarodinitril oder Cyclohexanon angewendet wird.

2. Verfahren nach Anspruch 1 zur Herstellung von 5-Nitroso-2,4,6-triaminopyrimidin durch Umsetzung von Malonsäuredinitril mit Guanidinhydrochlorid in Wasser bei einem pH unter 6,9 in Gegenwart von Natriumnitrit zum Guanidinsalz des Isonitrosomalonitrils, dadurch gekennzeichnet, dass die weitere Ueberführung dieses Salzes zum Endprodukt in Dimethylsulfoxid durch Wärmebehandlung erfolgt.